# EUROPEAN PATENT SPECIFICATION

(11) **EP 3 107 906 B1**
(45) Date of publication and mention of the grant of the patent: **05.12.2018**
(21) Application number: 15759564.6
(22) Date of filing: 19.02.2015
(51) Int. Cl.: C07D 333/38, A61K 31/381, A61P 27/06

(54) **REDUCED CENTRAL CORNEAL THICKENING BY USE OF HYDROPHILIC ESTER PRODRUGS OF BETA-CHLOROCYCLOPENTANES**
REDUZIERUNG DER VERDICKUNG DER ZENTRALEN HORNHAUT DURCH VERWENDUNG HYDROPHILER ESTER-PRODRUGS VON BETA-CHLORCYCLOPENTANEN
RÉDUCTION DE L'ÉPAISSISSEMENT CORNÉEN CENTRAL PAR UTILISATION DE PROMÉDICAMENTS D'ESTERS HYDROPHILES DE BÊTA-CHLOROCYCLOPENTANES

(30) Priority: 20.02.2014 US 201414185556
(43) Date of publication of application: 28.12.2016
(73) Proprietor: ALLERGAN, INC., Irvine, CA 92612 (US)
(72) Inventor: BURK, Robert M., Laguna Beach, California 92651 (US); IM, Wha Bin, Irvine, California 92620 (US)
(74) Representative: Hoffmann Eitle
(86) International application number: PCT/US2015/016660
(87) International publication number: WO 2015/175075

(56) References cited:
- WO-A1-2011/071620
- WO-A1-2011/091276
- WO-A1-2014/035827
- WO-A2-2008/008718
- US-A1- 2009 270 396

## Description

### FIELD OF THE INVENTION:

The present invention is directed to ester prodrugs and their use in lowering intraocular pressure and treating glaucoma.

### BACKGROUND OF THE INVENTION:

Glaucoma is one of the leading causes of blindness in the world, with over 2.5 million people in the United States suffering from the disease and several million more being at risk of developing glaucoma. As the population ages, the number of individuals suffering from glaucoma will continue to grow since the elderly are being affected disproportionally.

Based on its etiology, glaucoma can be classified into primary and secondary glaucoma. Primary glaucoma, also known as congenital glaucoma, can occur in the absence of other ocular conditions and its underlying causes are not known. However, it is known that increased intraocular pressure (IOP) observed in primary glaucoma is due to the obstruction of aqueous humor flow out of the eye. Secondary glaucoma results from another pre-existing ocular disease such as, uveitis, an intraocular tumor, enlarged cataract, central retinal vein occlusion, trauma to the eye, operative procedures and intraocular hemorrhage. Generally, any interference with the outward flow of aqueous humor from the posterior chamber into the anterior chamber and subsequently, into the canal of Schlemm can lead to secondary glaucoma.

Current treatments for glaucoma aim to reduce the pressure in the eye by decreasing the amount of aqueous fluid being produced or alternatively by enhancing the flow of fluid out of the eye by using mechanical means. Agents for topical application used to treat glaucoma include miotics (e.g. lsopto®Carpine, Ocusert®, Pilocar®, and Pilopine®) and epinephrines (e.g. Epifrin® and Propine®), which increase the outflow of fluid; beta blockers (e.g. Betagan®, Betimol®, Betoptic®, Ocupress®, Timoptic®, Optipranalol®), carbonic anhydrase inhibitors and alpha adrenergic agonists (e.g. Alphagan®, lopidine®, Trusopt®), which reduce the amount of fluid; and prostaglandin analogs (e.g. Lumigan®, Rescula®, Travatan®, Xalatan®), which increase the outflow of fluid through a secondary drainage route. WO 2008/008718 A2, US 2009/0270396 A1 and WO 2011/091276 A1 disclose other prostaglandin analogs.

The topical application of ophthalmic compositions for the treatment of glaucoma requires penetration of the drug through the cornea and into the anterior chamber, which contains aqueous humor, which then drains into the conventional outflow pathway. Intraocular pressure is lowered by drugs acting in the Schlemm's canal and the uveal-scleral pathway. Penetration of the drug through the cornea requires a balance of hydrophobic and hydrophilic characteristics. In order to diffuse into the cornea the drug must be sufficiently soluble in non-polar media and it must be sufficiently soluble in aqueous media in order to diffuse out of the cornea into the aqueous humor.

Potentially useful drugs for the treatment of glaucoma can be delivered as prodrug esters. The use of prodrug esters, which are cleaved enzymatically (e.g. in the cornea) to regenerate the active compound, can enhance penetration of the drug through the cornea into the anterior chamber. However, many esters are too hydrophobic to diffuse out of the relatively non-polar external layer of the cornea and into the aqueous humor. Further, such compounds are often not sufficiently soluble to formulate in aqueous solutions. There is a need in the art for ophthalmic compositions having the capability to penetrate through the cornea into the anterior chamber. At the same time such compositions need to exhibit sufficient hydrophilic properties to formulate in aqueous solution and to be soluble in the anterior chamber. Provided herein are compositions and methods addressing these and other needs in the art.

### SUMMARY OF THE INVENTION:

In one aspect, a compound selected from the group consisting of: 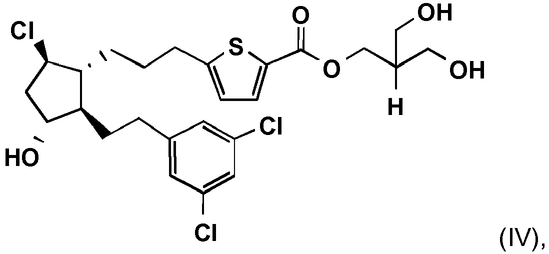 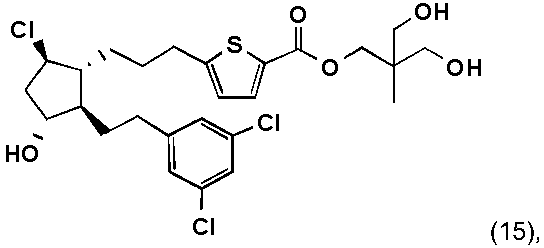 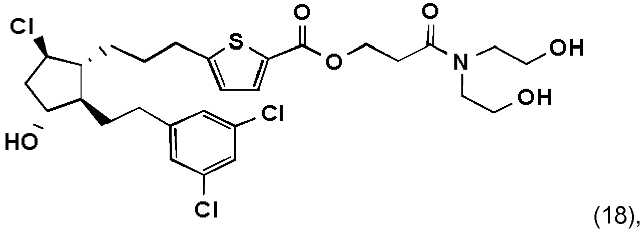 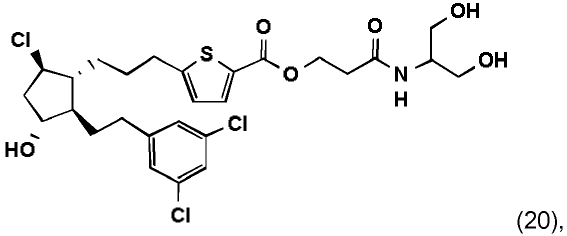 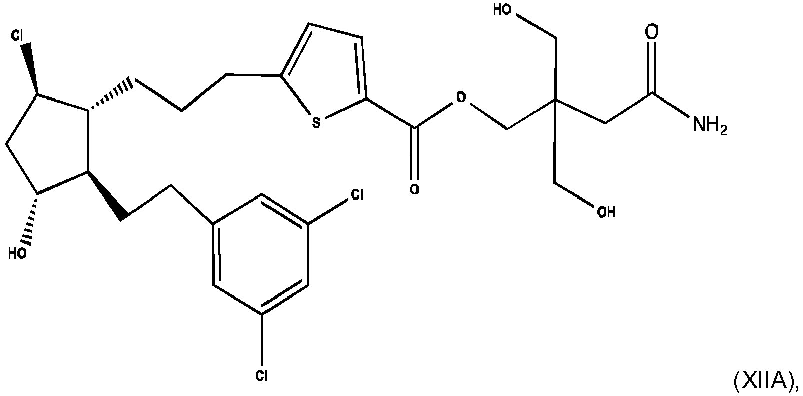 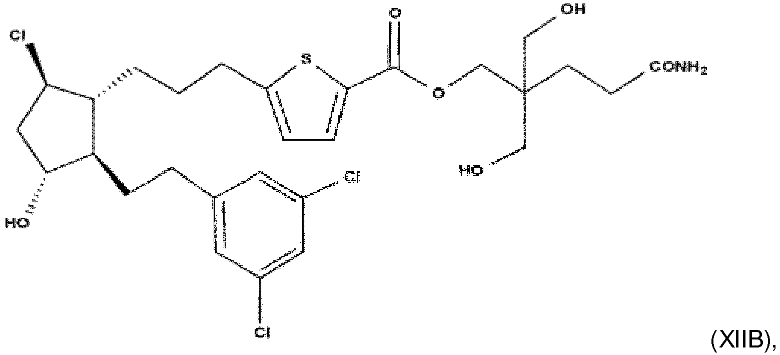 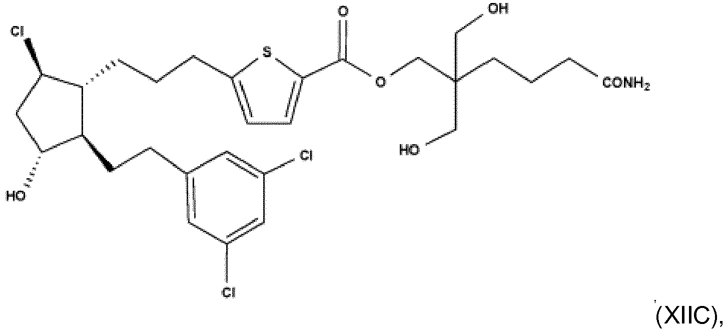 and 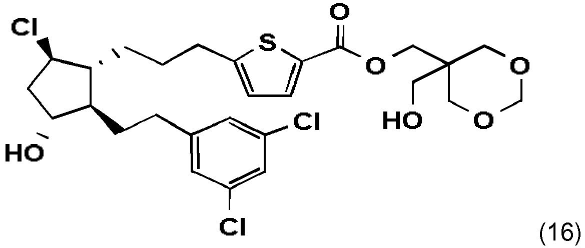 is provided.

In another aspect, an ophthalmic pharmaceutical composition including the compound provided herein and a pharmaceutically acceptable carrier is provided.

In another aspect, a compound or an ophthalmic composition provided herein for use in a method of treating an ophthalmic disease in a human is provided. The method includes administering a therapeutically effective amount of the compound provided herein to a subject in need thereof.

In another aspect, a compound or an ophthalmic composition provided herein for use in a method of reducing corneal thickening is provided. The method includes administering a therapeutically effective amount of the compound provided herein to a subject in need thereof.

### DETAILED DESCRIPTION OF THE INVENTION:

### I. Definitions

The abbreviations used herein have their conventional meaning within the chemical and biological arts. The chemical structures and formulae set forth herein are constructed according to the standard rules of chemical valency known in the chemical arts.

The compounds of the present invention may have asymmetric centers and/or geometric isomers. Compounds of the present invention containing an asymmetrically substituted atom may be isolated in optically active or racemic forms. It is well known in the art how to prepare optically active forms, such as by resolution of materials. All polymorphic forms, including amorphous form, and hydrates of a compound disclosed herein are within the scope of this invention.

Unless otherwise stated, structures depicted herein are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon are within the scope of this invention.

The compounds of the present invention may also contain unnatural proportions of atomic isotopes at one or more of atoms that constitute such compounds. For example, the compounds may be radiolabeled with radioactive isotopes, such as for example tritium (³H), iodine-125 (¹²⁵I) or carbon-14 (¹⁴C). All isotopic variations of the compounds of the present invention, whether radioactive or not, are encompassed within the scope of the present invention.

The terms "a," "an," or "a(n)," when used in reference to a group of substituents herein, mean at least one. For example, where a compound is substituted with "an" alkyl or aryl, the compound is optionally substituted with at least one alkyl and/or at least one aryl.

Descriptions of compounds of the present invention are limited by principles of chemical bonding known to those skilled in the art. Accordingly, where a group may be substituted by one or more of a number of substituents, such substitutions are selected so as to comply with principles of chemical bonding and to give compounds which are not inherently unstable and/or would be known to one of ordinary skill in the art as likely to be unstable under ambient conditions, such as aqueous, neutral, and several known physiological conditions. For example, a heterocycloalkyl or heteroaryl is attached to the remainder of the molecule via a ring heteroatom in compliance with principles of chemical bonding known to those skilled in the art thereby avoiding inherently unstable compounds.

The term "prodrug" is used according to its plain ordinary meaning and is intended to mean compounds that require a chemical or enzymatic transformation in order to release the active parent drug *in vivo* prior to producing a pharmacological effect.

A "pharmaceutically acceptable carrier" or "pharmaceutically acceptable excipient" means a carrier or an excipient that is useful in preparing a pharmaceutical composition that is generally safe, non-toxic and neither biologically nor otherwise undesirable, and includes a carrier or an excipient that is acceptable for veterinary use as well as human pharmaceutical use. "A pharmaceutically acceptable carrier/excipient" as used in the specification and claims includes both one and more than one such excipient.

The terms "treating" or "treatment" refers to any indicia of success in the treatment or amelioration of an injury, pathology or condition, including any objective or subjective parameter such as abatement; remission; diminishing of symptoms or making the injury, pathology or condition more tolerable to the patient; slowing in the rate of degeneration or decline; making the final point of degeneration less debilitating; improving a patient's physical or mental well-being. The treatment or amelioration of symptoms can be based on objective or subjective parameters; including the results of a physical examination, neuropsychiatric exams, and/or a psychiatric evaluation. For example, the certain methods presented herein successfully treat cancer by decreasing the incidence of cancer, in inhibiting its growth and/or causing remission of cancer.

An "effective amount" of a compound is an amount sufficient to contribute to the treatment, prevention, or reduction of a symptom or symptoms of a disease. Where recited in reference to a disease treatment, an "effective amount" may also be referred to as a "therapeutically effective amount." A "reduction" of a symptom or symptoms (and grammatical equivalents of this phrase) means decreasing of the severity or frequency of the symptom(s), or elimination of the symptom(s). A "prophylactically effective amount" of a drug is an amount of a drug that, when administered to a subject, will have the intended prophylactic effect, e.g., preventing or delaying the onset (or reoccurrence) a disease, disorder or condition, or reducing the likelihood of the onset (or reoccurrence) of a disease, disorder or condition or symptoms thereof. The full prophylactic effect does not necessarily occur by administration of one dose, and may occur only after administration of a series of doses. Thus, a prophylactically effective amount may be administered in one or more administrations.

The term "topical" in the context of methods described herein relates in the customary sense to the administration of a compound or pharmaceutical composition which is incorporated into a suitable pharmaceutical carrier and administered at a topical treatment site of a subject. Accordingly, the term "topical pharmaceutical composition" includes those pharmaceutical forms in which the compound is administered externally by direct contact with a topical treatment site, e.g., the eye or the skin. The term "topical ocular pharmaceutical composition" refers to a pharmaceutical composition suitable for administering directly to the eye. The term "topical epidermal pharmaceutical composition" refers to a pharmaceutical composition suitable for administering directed to the epidermal layer of the skin, e.g., the palpebra, the supercilium, the scalp, or the body. The term "topical administering" refers to administering externally by direct contact with a topical treatment site. The term "topical epidermal administering" refers to administering externally by direct contact with the epidermis. The term "topical ocular administering" refers to administering externally by direct contact with the eye.

### II. Compositions

In one embodiment, the compound has the structure of formula: 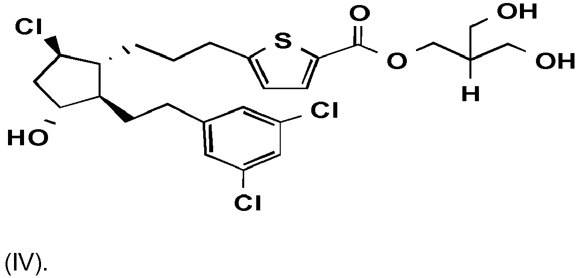 In one embodiment the compound has the structure:

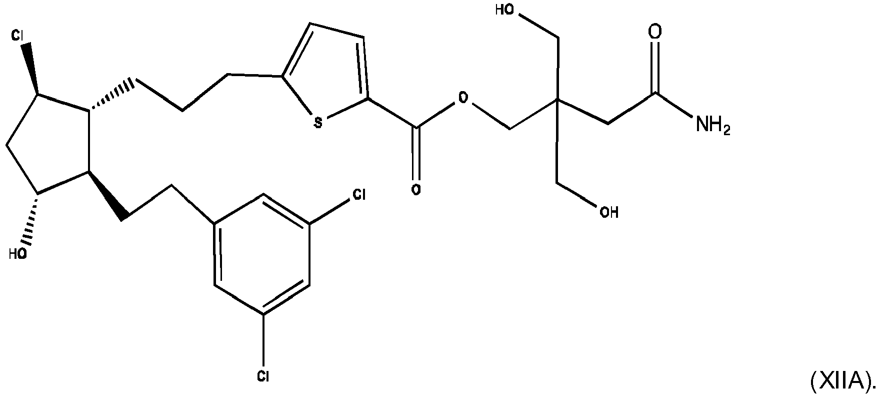

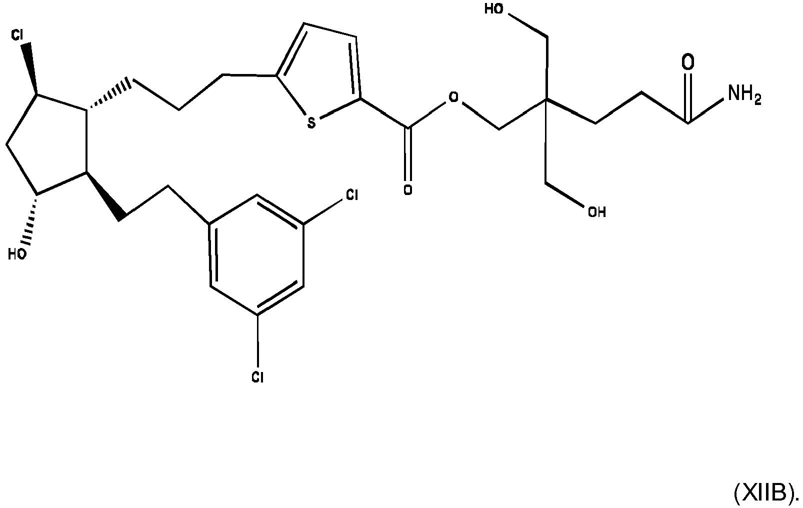

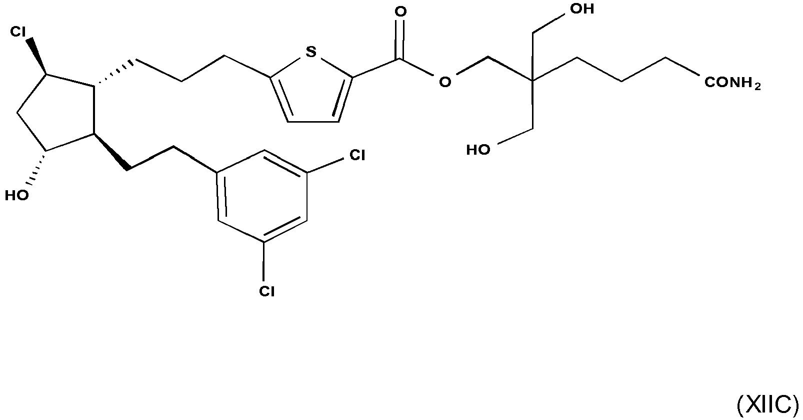

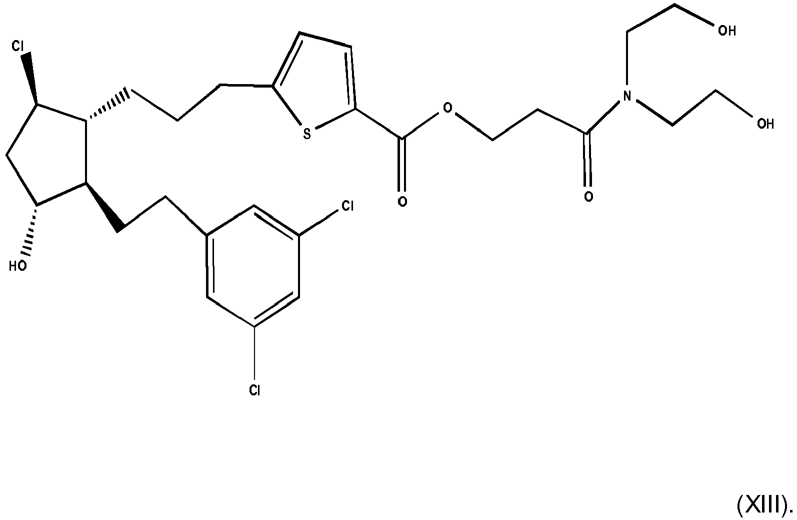

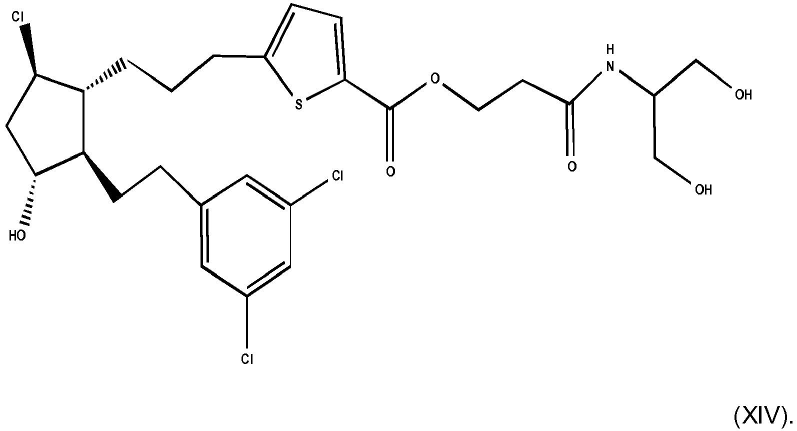

### III. Pharmaceutical Compositions:

In another aspect, an ophthalmic pharmaceutical composition including a compound provided herein (e.g., a compound of formulae IV, XIIA - XIV) is provided. In one embodiment, the compound has the structure of formula (IV). In one embodiment, the compound has the structure of one of formulae XII, XIII or XIV.

In one embodiment, the pharmaceutical composition is a solution, emulsion, gel or foam. In one embodiment, the pharmaceutical composition is a solution. In one embodiment, the pharmaceutical composition is an emulsion. In one embodiment, the pharmaceutical composition is a gel. In one embodiment, the pharmaceutical composition is a foam.

### A. Formulations:

The compounds and pharmaceutical compositions disclosed herein can be prepared and administered in a variety of forms including solution, emulsion, gel or foam. Accordingly, pharmaceutical compositions contemplated herein include a pharmaceutically acceptable carrier or excipient and one or more compounds described herein. "Solution" refers in the customary sense to a liquid pharmaceutical composition in which a compound (e.g., a compound described herein), is at least partially dissolved, preferably fully dissolved, and which can be administered as a liquid. "Emulsion" refers in the customary sense to a mixture of two or more immiscible liquids, one compound (e.g., a compound described herein or solution thereof) being dispersed through the other compound (e.g., a carrier as described herein). "Gel" refers in the customary sense to a highly viscous solution, emulsion, or colloidal suspension of a compound within a continuous fluid phase resulting in a viscous semirigid fluid. "Colloid" refers in the customary sense to a composition which includes a continuous medium throughout which are distributed small particles which do not settle under the influence of gravity. "Foam" refers in the customary sense to a composition which includes a continuous medium (i.e., solution, emulsion, gel and the like) through which gas (e.g., air) is dispersed.

Pharmaceutical compositions contemplated herein may be prepared by combining a therapeutically effective amount of at least one compound as described herein as an active ingredient in combination with one or more conventional pharmaceutically acceptable excipients, and by preparation of unit dosage forms suitable for topical use. The therapeutically efficient amount typically is between about 0.0001 and about 5% (w/v), preferably about 0.001 to about 1.0% (w/v) in liquid formulations which include solutions, emulsions, gels and foams. Pharmaceutical admixtures suitable for use in the present invention include those described, for example, in PHARMACEUTICAL SCIENCES (17th Ed., Mack Pub. Co., Easton, PA) and WO 96/05309.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

Some compounds may have limited solubility in water and therefore may require a surfactant or other appropriate co-solvent in the composition. Such co-solvents include: Polysorbate 20, 60, and 80; Pluronic F-68, F-84, and P-103; cyclodextrin; and polyoxyl 35 castor oil. Such co-solvents are typically employed at a level between about 0.01 % and about 2% by weight.

Viscosity greater than that of simple aqueous solutions may be desirable to decrease variability in dispensing the formulations, to decrease physical separation of components of a suspension or emulsion of formulation, and/or otherwise to improve the formulation. Such viscosity building agents include, for example, polyvinyl alcohol, polyvinyl pyrrolidone, methyl cellulose, hydroxy propyl methylcellulose, hydroxyethyl cellulose, carboxymethyl cellulose, hydroxy propyl cellulose, chondroitin sulfate and salts thereof, hyaluronic acid and salts thereof, and combinations of the foregoing. Such agents are typically employed at a level between about 0.01% and about 2% by weight.

The compositions of the present invention may additionally include components to provide sustained release and/or comfort. Such components include high molecular weight, anionic mucomimetic polymers, gelling polysaccharides, and finely-divided drug carrier substrates. These components are discussed in greater detail in U.S. Pat. Nos. 4,911,920; 5,403,841; 5,212,162; and 4,861,760, US Patent application publication No. US 2011-0124736 A1, also corresponding to US Patent application serial no. 12/940,711.

For ophthalmic application, preferably solutions are prepared using a physiological saline solution as a major vehicle. The pH of such ophthalmic solutions should preferably be maintained between 4.5 and 8.0 with an appropriate buffer system, a neutral pH being preferred but not essential. The formulations may also contain conventional, pharmaceutically acceptable preservatives, stabilizers and surfactants.

Various buffers and means for adjusting pH may be used so long as the resulting preparation is ophthalmically acceptable. Accordingly, buffers include acetate buffers, citrate buffers, phosphate buffers and borate buffers. Acids or bases may be used to adjust the pH of these formulations as needed.

Preferred preservatives that may be used in the pharmaceutical compositions of the present invention include, but are not limited to, benzalkonium chloride, chlorobutanol, thimerosal, phenylmercuric acetate and phenylmercuric nitrate. A preferred surfactant is, for example, Tween 80. Likewise, various preferred vehicles may be used in the ophthalmic preparations of the present invention. These vehicles include, but are not limited to, polyvinyl alcohol, povidone, hydroxypropyl methyl cellulose, poloxamers, carboxymethyl cellulose, hydroxyethyl cellulose cyclodextrin and purified water.

Tonicity agents may be added as needed or convenient. They include, but are not limited to, salts, particularly sodium chloride, potassium chloride, mannitol and glycerin, or any other suitable ophthalmically acceptable tonicity adjustor.

An ophthalmically acceptable antioxidant for use in the present invention includes, but is not limited to, sodium metabisulfite, sodium thiosulfate, acetylcysteine, butylated hydroxyanisole and butylated hydroxytoluene.

Other excipient components which may be included in the ophthalmic preparations are chelating agents. The preferred chelating agent is edentate disodium, although other chelating agents may also be used in place of or in conjunction with it.

The ophthalmic formulations of the present invention are conveniently packaged in forms suitable for metered application, such as in containers equipped with an orifice, to facilitate application to the eye. Vials suitable for unit dose application are usually made of suitable inert, non-toxic plastic material, and generally contain between about 0.5 and about 15 ml solution, emulsion, gel or foam. One package may contain one or more unit doses.

Preservative-free solutions are often formulated in non-resealable containers containing up to about ten, preferably up to about five units doses, where a typical unit dose is from one to about 8 drops, preferably one to about 3 drops.

Typically, the compounds are applied repeatedly for a sustained period of time topically on the part of the body to be treated, for example, the eye. The preferred dosage regimen will generally involve regular administration for a period of treatment of at least one month, more preferably at least three months, and most preferably at least six months. The regular administration can be 1, 2, 3, 4 or even more times per day.

### IV. Methods of Treatment:

In another aspect, the compounds or compositions disclosed herein for use in a method of treating an ophthalmic disease in a human are provided. The method includes administering a therapeutically effective amount of a compound provided herein and embodiments thereof (e.g., a compound of formula IV, XIIA - XIV) to a subject in need thereof. In one embodiment, the administering is topical administering. In one embodiment, the disease is macular degeneration. In one embodiment, the disease results from intraocular pressure. In one embodiment, the disease is glaucoma.

In one embodiment, a combination treatment with a β-blocker (or β-adrenergic antagonist) including carteolol, levobunolol, metiparanolol, timolol hemihydrate, timolol maleate, or β1-selective antagonists such as betaxolol.

In one embodiment, a combination treatment with an adrenergic agonists including non-selective adrenergic agonists such as epinephrine borate, epinephrine hydrochloride, and dipivefrin, and the like, or an α2-selective adrenergic agonists such as apraclonidine, or brimonidine.

In one embodiment, a combination treatment with a carbonic anhydrase inhibitors including acetazolamide, dichlorphenamide, methazolamide, brinzolamide, dorzolamide, and the like, is provided.

In one embodiment, a combination treatment with a cholinergic agonist including direct acting cholinergic agonists such as carbachol, pilocarpine hydrochloride, pilocarpine nitrate, pilocarpine, and the like, is provided.

In one embodiment, a combination treatment with a cholinesterase inhibitors such as demecarium, echothiophate, physostigmine, and the like, is provided.

In one embodiment, a combination treatment with a glutamate antagonists or other neuroprotective agents such as Ca²⁺ channel blockers such as memantine, amantadine, rimantadine, nitroglycerin, dextrorphan, dextromethorphan, CGS-19755, dihydropyridines, verapamil, emopamil, benzothiazepines, bepridil, diphenylbutylpiperidines, diphenylpiperazines, HOE 166 and related drugs, fluspirilene, eliprodil, ifenprodil, CP-1 01,606, tibalosine, 2309BT, and 840S, flunarizine, nicardipine, nifedipine, nimodipine, bamidipine, verapamil, lidoflazine, prenylamine lactate, amiloride, and the like, is provided.

In one embodiment, a combination treatment with prostamides such as bimatoprost, is provided.

In one embodiment, a combination treatment with a prostaglandin including travoprost, UF0-21, chloprostenol, fluprostenol, 13,14-dihydro-chloprostenol, isopropyl unoprostone, latanoprost and the like is provided.

In one embodiment, a combination treatment with a cannabinoid including CB1 agonists such as WIN-55212-2 and CP-55940 and the like, is provided.

In another aspect, the compounds and compositions disclosed herein for use in a method of reducing corneal thickening are provided. The method includes administering a therapeutically effective amount of a compound provided herein and embodiments thereof (e.g., a compound of formula IV, XIIA - XIV) to a subject in need thereof. In one embodiment, the subject suffers from glaucoma. In one embodiment, the subject suffers from ocular hypertension.

In one embodiment, a method of using a compound disclosed herein for the manufacture of a medicament for the treatment of glaucoma or ocular hypertension is provided. In one embodiment, the compound has the structure of any one of formulae IV, XIIA - XIV. In one embodiment, the compound has the structure of one of formulae (Xlla)-(Xllc). In one embodiment, the compound has the structure of formula (XIII). In one embodiment, the compound has the structure of one of formulae (XIV).

### V. Examples:

Abbreviations used herein have the customary meaning in the chemical arts. Specific abbreviations include the following: TBDMSO: (tert-butyldimethylsilyl)oxy; DMF: dimethylformamide; EDC: 1-ethyl-3-[3-dimethylaminopropyl]carbodiimide hydrochloride; DMAP: 4-dimethylaminopyridine; THF: tetrahydrofuran; Bu₄NF: tetrabutylammonium fluoride.

### Example 12. Synthesis of 3-Hydroxy-2-(hydroxymethyl)propyl 5-(3-((1R,2R,3R,5R)-5-chloro-2-(3,5-dichlorophenethyl)-3-hydroxycyclopentyl)propyl)thiophene-2-carboxylate (compound (IV)).

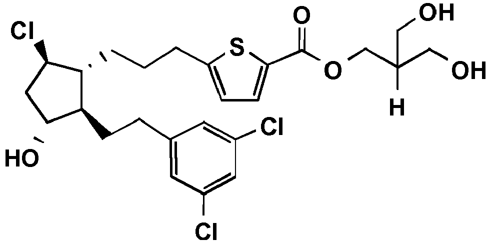

(2,2-Dimethyl-1,3-dioxan-5-yl)methanol (126.6 mg, 0.866 mmol) was added to a solution of the 5-(3-((1R,2R,3R,5R)-5-chloro-2-(3,5-dichlorophenethyl)-3-hydroxycyclopentyl)propyl)thiophene-2-carboxylic acid **1** (200mg, 0.433mmol), 4-(dimethylamino)pyridine (55.3 mg, 0.453 mmol) and N-(3-dimethylaminopropyl)-N'-ethylcarbodiimide hydrochloride (91.3 mg, 0.476 mmol) in DMF (3.0 mL) at 23 °C. After stirring for 48h the reaction solution was diluted with EtOAc and washed with 1N HCI, saturated aqueous NaHCO₃ then brine. The organic portion was dried (MgSO₄), filtered and concentrated *in vacuo* to provide a clear, viscous oil.

The ester obtained above was stirred in a mixture of 1N HCl:THF (1:1, 4mL) at 23 °C for 16h. The reaction mixture was diluted with EtOAc and was with water, saturated aqueous NaHCO₃ then brine. The organic portion was dried (MgSO₄), filtered and concentrated *in vacuo.* Purification of the residue by flash column chromatography (silica gel, 1:1 hex/EtOAc followed by 100% EtOAc) afforded 57.0 mg (24%) of pure dihydroxyester (IV) as a clear, colorless oil.

### Example 13. Synthesis of 3-Hydroxy-2-(hydroxymethyl)-2-methylpropyl 5-(3-((1R,2R,3R,5R)-5-chloro-2-(3,5-dichlorophenethyl)-3-hydroxycyclopentyl)propyl)thiophene-2-carboxylate (compound 15).

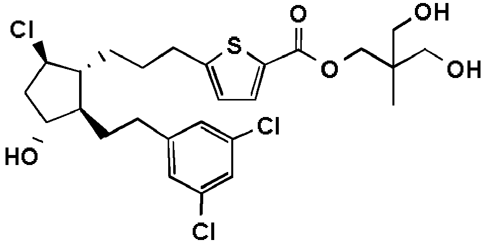

5-Hydroxymethyl-2,2,5-trimethyl-1,3-dioxane (0.26 mL, 1.73 mmol) was added to a solution of the 5-(3-((1R,2R,3R,5R)-5-chloro-2-(3,5-dichlorophenethyl)-3-hydroxycyclopentyl)propyl)thiophene-2-carboxylic acid **1** (200mg, 0.433mmol), 4-(dimethylamino)pyridine (55.3 mg, 0.453 mmol) and N-(3-dimethylaminopropyl)-N'-ethylcarbodiimide hydrochloride (91.3 mg, 0.476 mmol) in DMF (3.0 mL) at 23 °C. After stirring for 48h the reaction solution was diluted with EtOAc and washed with 1N HCI, saturated aqueous NaHCO₃ then brine. The organic portion was dried (MgSO₄), filtered and concentrated *in vacuo* to provide a clear, viscous oil.

The ester obtained above was stirred in a mixture of 1N HCl:THF (1:1, 4mL) at 23 °C for 16h. The reaction mixture was diluted with EtOAc and was with water, saturated aqueous NaHCO₃ then brine. The organic portion was dried (MgSO₄), filtered and concentrated *in vacuo.* Purification of the residue by flash column chromatography (silica gel, 1:1 hex/EtOAc followed by 100% EtOAc) afforded 22.4 mg (9%) of pure dihydroxyester **15** as a clear, colorless oil.

### Example 14. Synthesis of (5-(Hydroxymethyl)-1,3-dioxan-5-yl)methyl 5-(3-((1R,2R,3R,5R)-5-chloro-2-(3,5-dichlorophenethyl)-3-hydroxycyclopentyl)propyl)thiophene-2-carboxylate (compound 16).

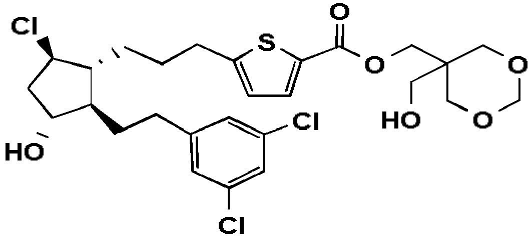

(1,3-dioxane-5,5-diyl)dimethanol (256.6 mg, 1.73 mmol) was added to a solution of the 5-(3-((1R,2R,3R,5R)-5-chloro-2-(3,5-dichlorophenethyl)-3-hydroxycyclopentyl)propyl)thiophene-2-carboxylic acid **1** (200mg, 0.433mmol), 4-(dimethylamino)pyridine (55.3 mg, 0.453 mmol) and N-(3-dimethylaminopropyl)-N'-ethylcarbodiimide hydrochloride (91.3 mg, 0.476 mmol) in DMF (3.0 mL) at 23 °C. After stirring for 48h the reaction solution was diluted with EtOAc and washed with 1N HCI, saturated aqueous NaHCO₃ then brine. The organic portion was dried (MgSO₄), filtered and concentrated *in vacuo* Purification of the residue by flash column chromatography (silica gel, 2:1 hex/EtOAc followed by , 1:1 hex/EtOAc) afforded 205.7 mg (80%) of pure hydroxymethylester **16** as a clear, colorless oil.

### Example 15. Synthesis of 3-(bis(2-((tetrahydro-2H-pyran-2-yl)oxy)ethyl)amino)-3-oxopropyl 5-(3-((1R,2R,3R,5R)-5-chloro-2-(3,5-dichlorophenethyl)-3-hydroxycyclopentyl)propyl)thiophene-2-carboxylate (compound 17).

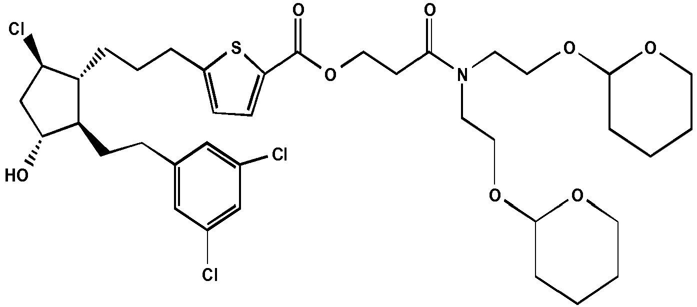

N-(3-dimethylaminopropyl)-N'-ethylcarbodiimide hydrochloride (91.3 mg, 0.476 mmol) was added to a solution of the 5-(3-((1R,2R,3R,5R)-5-chloro-2-(3,5-dichlorophenethyl)-3-hydroxycyclopentyl)propyl)thiophene-2-carboxylic acid **1** (200 mg, 0.433 mmol), 4-(dimethylamino)pyridine (55.3 mg, 0.453 mmol) and 3-hydroxy-N,N-bis(2-((tetrahydro-2H-pyran-2-yl)oxy)ethyl)propanamide (597.6 mg, 1.73 mmol) in DMF (4.5 mL) at 23 °C. After stirring for 48h the reaction was diluted with EtOAc and washed with 1N HCI, saturated aqueous NaHCO₃ then brine. The organic portion was dried (MgSO₄), filtered and concentrated *in vacuo.* Flash column chromatography (silica gel, 1:1 hex/EtOAc) afforded 120.4 mg (35%) of pure ester **17** as a clear, colorless oil.

### Example 16. Synthesis of 3-(Bis(2-hydroxyethyl)amino)-3-oxopropyl 5-(3-((1R,2R,3R,5R)-5-chloro-2-(3,5-dichlorophenethyl)-3-hydroxycyclopentyl)propyl)thiophene-2-carboxylate (compound 18).

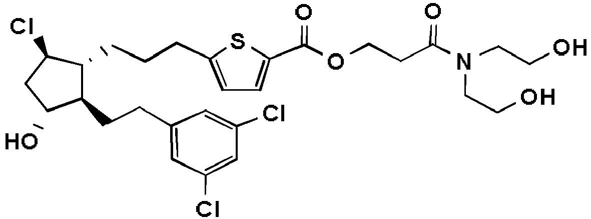

Pyridinium p-toluenesulfonate (38.2 mg, 0.152 mmol) was added to a solution of the ester **17** (120 mg, 0.152 mmol) in MeOH (3.0 mL). The reaction was allowed to stir at 23 °C for 48h. The solvent was then removed *in vacuo.* The residue was diluted with EtOAc and washed with 1N HCI, saturated aqueous NaHCO₃ then brine. The organic portion was dried (MgSO₄), filtered and concentrated *in vacuo.* Purification of the residue by flash column chromatography (silica gel, 100% EtOAc followed by 39:1 EtOAc/MeOH) afforded 54.0 mg (57%) of pure dihydroxyester **18** as a clear, colorless oil.

### Example 17. Synthesis of 3-((1,3-bis((tetrahydro-2H-pyran-2-yl)oxy)propan-2-yl)amino)-3-oxopropyl 5-(3-((1R,2R,3R,5R)-5-chloro-2-(3,5-dichlorophenethyl)-3-hydroxycyclopentyl)-propyl)thiophene-2-carboxylate (compound19).

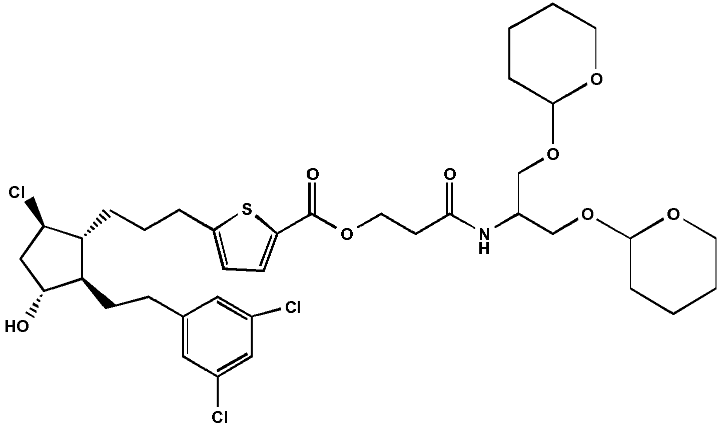

N-(3-dimethylaminopropyl)-N'-ethylcarbodiimide hydrochloride (91.3 mg, 0.476 mmol) was added to a solution of the 5-(3-((1 R,2R,3R,5R)-5-chloro-2-(3,5-dichlorophenethyl)-3-hydroxycyclopentyl)propyl)thiophene-2-carboxylic acid **1** (200 mg, 0.433 mmol), 4-(dimethylamino)pyridine (55.3 mg, 0.453 mmol) and N-(1,3-bis((tetrahydro-2H-pyran-2-yl)oxy)propan-2-yl)-3-hydroxypropanamide (574 mg, 1.73 mmol) in DMF (4.5 mL) at 23 °C. After stirring for 48h the reaction was diluted with EtOAc and washed with 1N HCI, saturated aqueous NaHCO₃ then brine. The organic portion was dried (MgSO₄), filtered and concentrated *in vacuo.* Flash column chromatography (silica gel, 1:1 hex/EtOAc) afforded 60.7 mg (18%) of pure ester **19** as a clear, colorless oil.

### Example 18. Synthesis of 3-((1,3-Dihydroxypropan-2-yl)amino)-3-oxopropyl 5-(3-((1R,2R,3R,5R)-5-chloro-2-(3,5-dichlorophenethyl)-3-hydroxycyclopentyl)propyl)thiophene-2-carboxylate (compound 20).

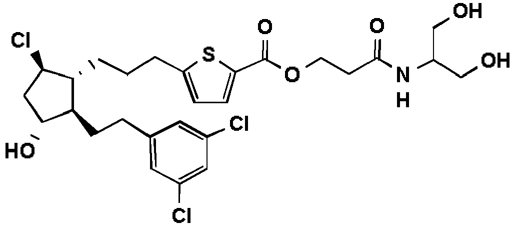

Pyridinium p-toluenesulfonate (19.6 mg, 0.078 mmol) was added to a solution of the ester **19** (60.7 mg, 0.078 mmol) in MeOH (3.0 mL). The reaction was allowed to stir at 23 °C for 48h. The solvent was then removed *in vacuo.* The residue was diluted with EtOAc and washed with 1N HCI, saturated aqueous NaHCO₃ then brine. The organic portion was dried (MgSO₄), filtered and concentrated *in vacuo.* Purification of the residue by flash column chromatography (silica gel, 100% EtOAc followed by 19:1 EtOAc/MeOH) afforded 37.2 mg (79%) of pure dihydroxyester **20** as a clear, colorless oil.

## Claims

1. A compound selected from the group consisting of: 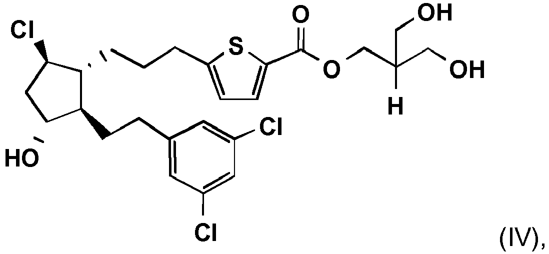 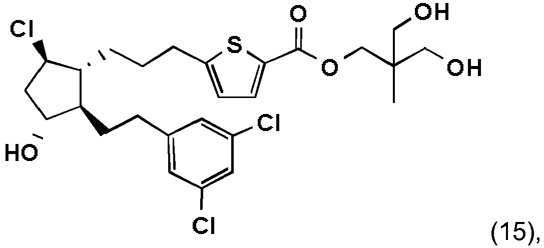 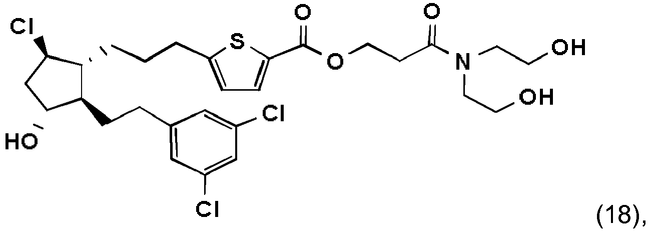 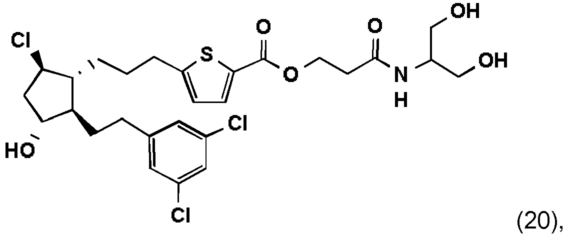 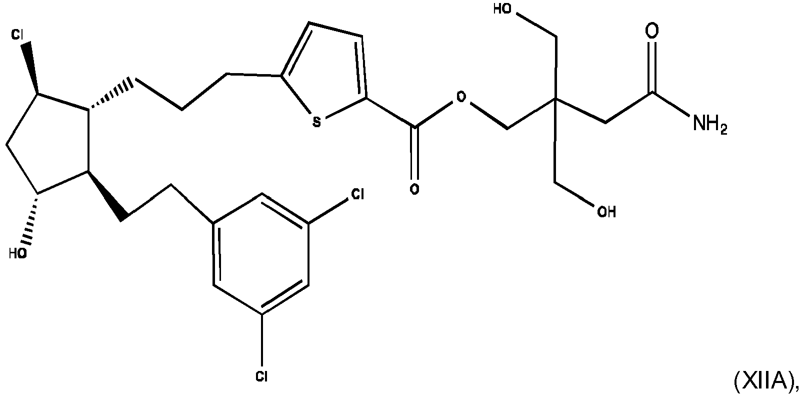 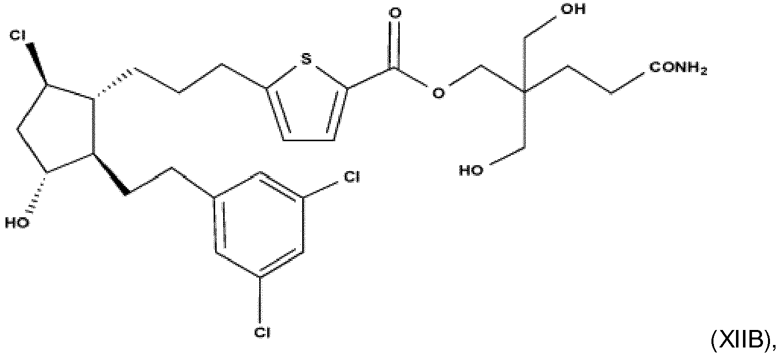 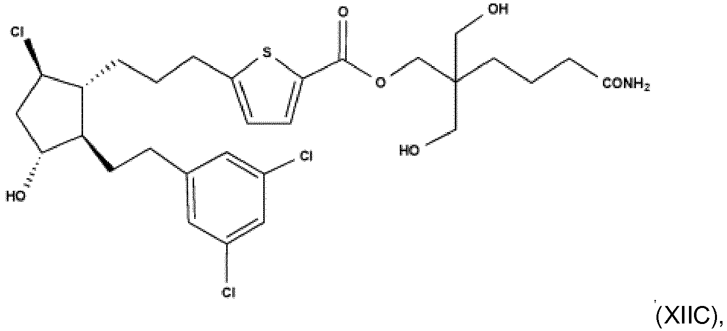 and

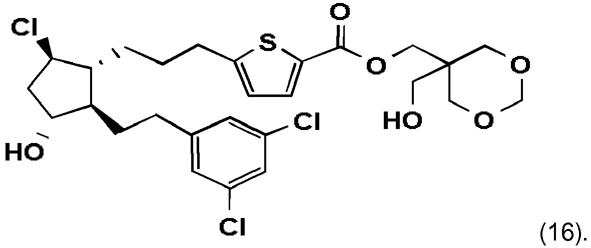

2. A pharmaceutical composition comprising the compound of claim 1 and a pharmaceutically acceptable carrier.

3. The compound of claim 1 or the pharmaceutical composition of claim 2 for use as medicament.

4. The compound of claim 1 or the pharmaceutical composition of claim 2 for use in a method of treating an ophthalmic disease in a human, wherein the method includes administering a therapeutically effective amount of said compound or said pharmaceutical composition to a subject in need thereof.

5. The compound of claim 1 or the pharmaceutical composition of claim 2 for use in a method of reducing corneal thickening, wherein the method includes administering a therapeutically effective amount of said compound or said pharmaceutical composition to a subject in need thereof.

## Patentansprüche

1. Verbindung, ausgewählt aus der Gruppe bestehend aus:

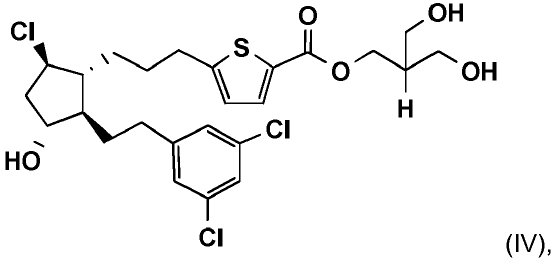

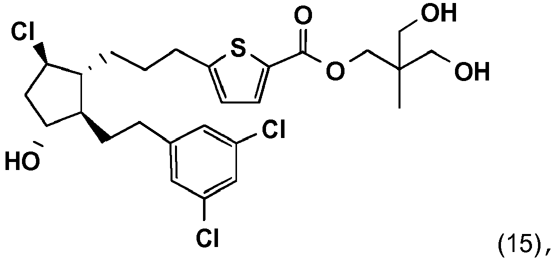

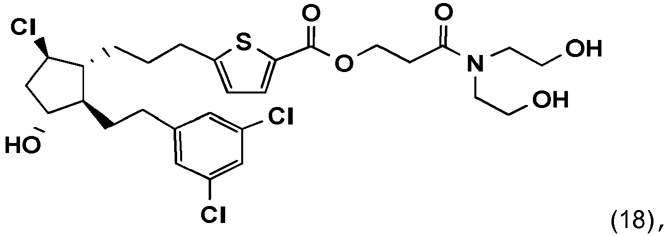

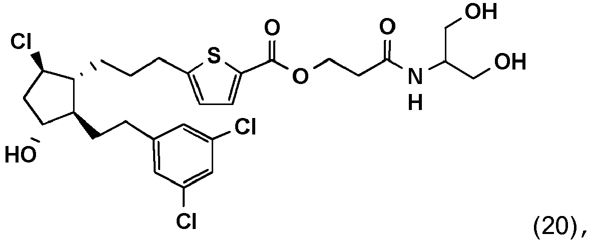

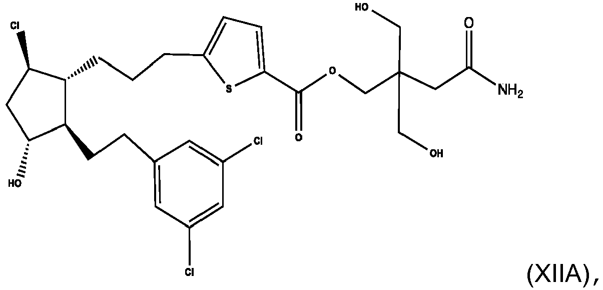

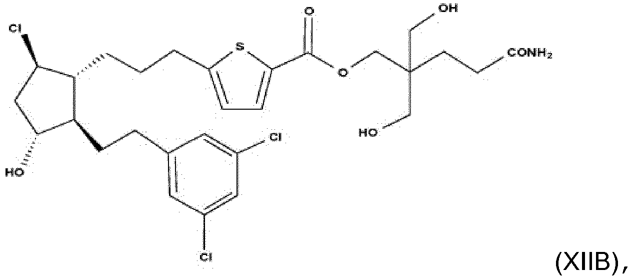

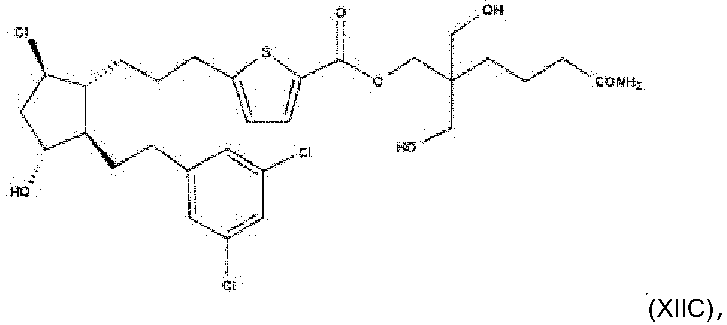

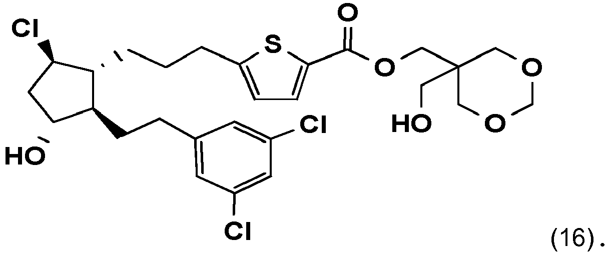

2. Pharmazeutische Zusammensetzung, umfassend die Verbindung gemäss Anspruch 1 und einen pharmazeutisch annehmbaren Träger.

3. Verbindung gemäss Anspruch 1 oder pharmazeutische Zusammensetzung gemäss Anspruch 2 zur Verwendung als Arzneimittel.

4. Verbindung gemäss Anspruch 1 oder pharmazeutische Zusammensetzung gemäss Anspruch 2 zur Verwendung in einem Verfahren zur Behandlung einer Augenkrankheit bei einem Menschen, wobei das Verfahren die Verabreichung einer therapeutisch wirksamen Menge dieser Verbindung oder dieser pharmazeutischen Zusammensetzung an ein Individuum, das diese benötigt, einschliesst.

5. Verbindung gemäss Anspruch 1 oder pharmazeutische Zusammensetzung gemäss Anspruch 2 zur Verwendung in einem Verfahren zur Reduzierung der Hornhautverdickung, wobei das Verfahren die Verabreichung einer therapeutisch wirksamen Menge dieser Verbindung oder dieser pharmazeutischen Zusammensetzung an ein Individuum, das diese benötigt, einschliesst.

## Revendications

1. Composé choisi parmi le groupe constitué de : 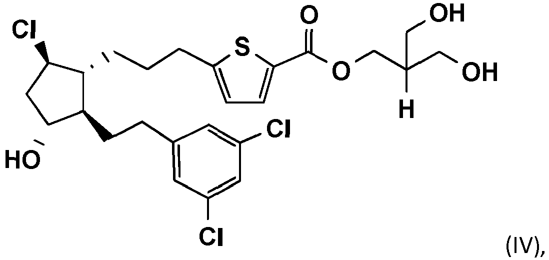 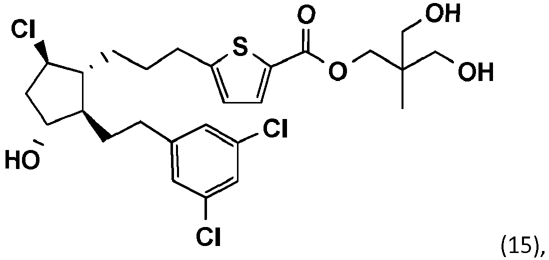 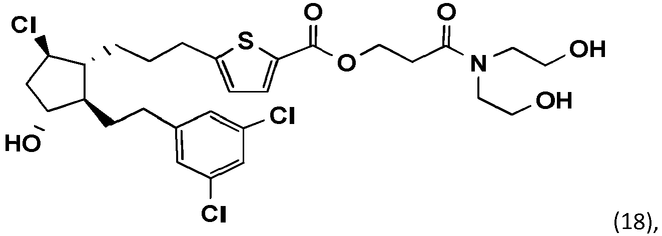 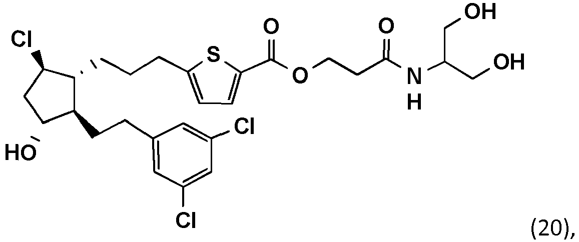 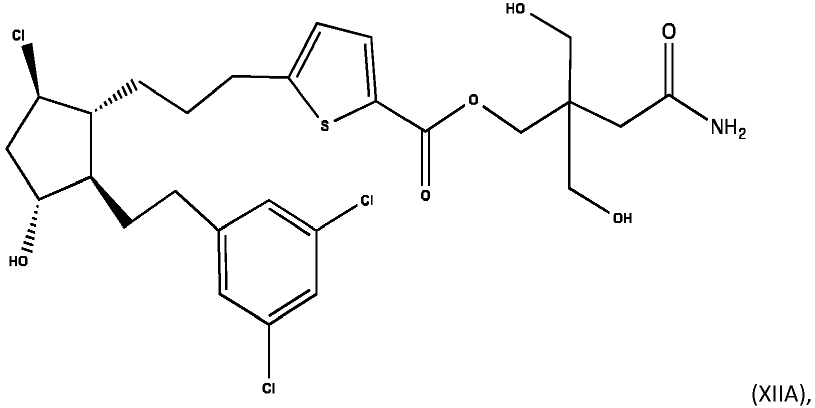 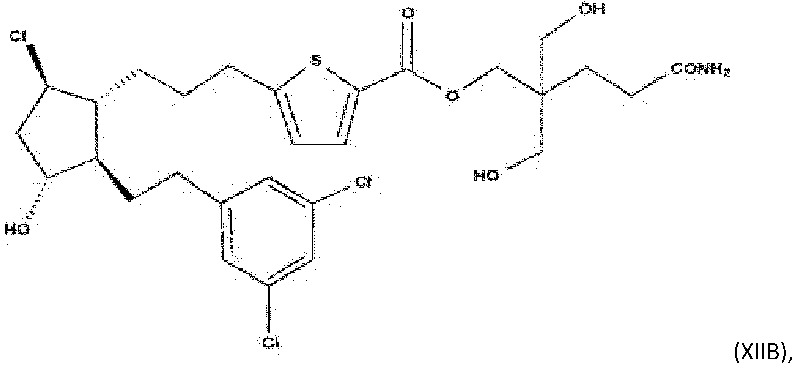 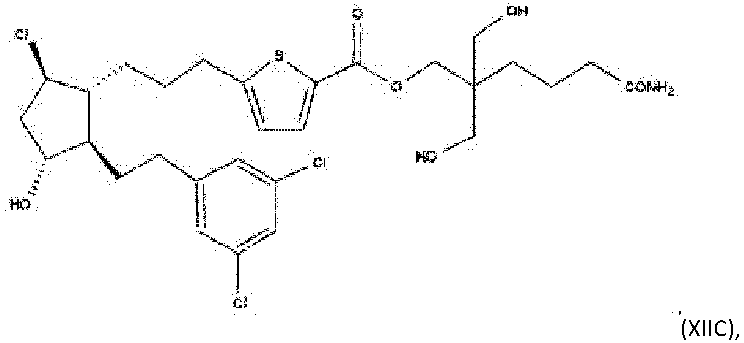 et

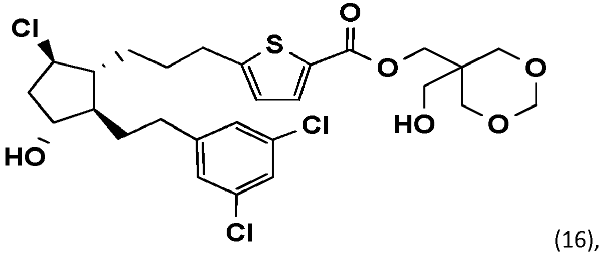

2. Composition pharmaceutique comprenant le composé de la revendication 1 et un véhicule pharmaceutiquement acceptable.

3. Le composé de la revendication 1 ou la composition pharmaceutique de la revendication 2 pour l'utilisation en tant que médicament.

4. Le composé de la revendication 1 ou la composition pharmaceutique de la revendication 2 pour l'utilisation dans une méthode de traitement d'une maladie ophtalmique dans un humain, laquelle méthode comporte l'administration d'une quantité thérapeutiquement efficace dudit composé ou de ladite composition pharmaceutique à un sujet qui en a besoin.

5. Le composé de la revendication 1 ou la composition pharmaceutique de la revendication 2 pour l'utilisation dans une méthode de réduction l'épaississement de la cornée, laquelle méthode comporte l'administration d'une quantité thérapeutiquement efficace dudit composé ou de ladite composition pharmaceutique à un sujet qui en a besoin.
